(19) Europäisches Patentamt
European Patent Office
Office européen des brevets

(11) Veröffentlichungsnummer: **0 433 928 B1**

(12) **EUROPÄISCHE PATENTSCHRIFT**

(45) Veröffentlichungstag der Patentschrift: **15.03.95**

(21) Anmeldenummer: **90124305.5**

(22) Anmeldetag: **15.12.90**

(51) Int. Cl.⁶: **C07F 9/40**, A61K 31/66, C07F 9/38, C07F 9/6558, C07F 9/6571, C07F 9/653, C07F 9/6539, C07F 9/58

# (54) 2-Formylbenzylphosphonsäure-Derivate, deren Herstellung und ihre Verwendung zur Behandlung von durch Viren verursachte Krankheiten.

(30) Priorität: **21.12.89 DE 3942318**

(43) Veröffentlichungstag der Anmeldung:
**26.06.91 Patentblatt 91/26**

(45) Bekanntmachung des Hinweises auf die Patenterteilung:
**15.03.95 Patentblatt 95/11**

(84) Benannte Vertragsstaaten:
**AT BE CH DE DK ES FR GB GR IT LI LU NL SE**

(56) Entgegenhaltungen:
**EP-A- 0 313 002**

(73) Patentinhaber: **HOECHST AKTIENGESELL-SCHAFT**

**D-65926 Frankfurt (DE)**

(72) Erfinder: **Peyman, Anuschirwan, Dr.**
**In den Bleichwiesen 9**
**W-6233 Kelkheim (Taunus) (DE)**
Erfinder: **Uhlmann, Eugen, Dr.**
**Zum Talblick 31**
**W-6246 Glashütten/Taunus (DE)**
Erfinder: **Winkler, Irwin, Dr.**
**In den Eichen 40**
**W-6237 Liederbach (DE)**
Erfinder: **Helsberg, Matthias, Dr.**
**Am Rosengarten 3**
**W-6233 Kelkheim/Taunus (DE)**
Erfinder: **Meichsner, Christoph, Dr.**
**Bienerstrasse 30**
**W-6238 Hofheim am Taunus (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die Erfindung betrifft neue 2-Formylbenzylphosphonsäure-Derivate, Verfahren zur Herstellung dieser Verbindungen, pharmazeutische Mittel, welche die erfindungsgemäßen aktiven Verbindungen enthalten und deren Verwendung als Arzneimittel, insbesondere zur Behandlung von durch Viren verursachte Krankheiten.

Zur Behandlung von durch Viren verursachte Krankheiten wurden bisher verschiedene Präparate eingesetzt, wie z. B. Nukleosidanaloga, Amantadin, Pyrophosphatanaloga oder Immunmodulatoren (M.J. Wood, A.M. Geddes, The Lancet, 1987, 1189). Es sind einige Phosphonsäure-Derivate bekannt, die antivirale Aktivität zeigen. Dazu gehören Verbindungen wie Phosphonoameisensäure (PFA), Phosphonoessigsäure (PAA), Methylendiphosphonsäure (MDP) sowie Tetrazolphosphonsäuren (S.M. Roberts, NATO ASI Ser., Ser. A 143, 1988, 37; D.W. Hutchinson, M. Naylor, Nucleic Acids Res., 13, 1985, 8519). PFA hat ein breites antivirales Spektrum, verursacht aber einige toxische Nebeneffekte, die eine Entwicklung zum antiviralen Medikament bisher behinderten (M.J. Wood, A.M. Geddes, The Lancet, 1987, 1189). Von ortho-Phosphonyloxy-acetophenon-Derivaten ist bekannt, daß sie insbesondere gegen Picorna-Viren wirken (EP 21 000).

Diana et al. (J. Med. Chem. 27, 1984, 691; DOS 29 22 054) berichten über eine Verbindungsklasse des Typs

A----------C

B

in der A ein aromatischer Ring und C ein Phosphonat oder ein $\beta$-Keto-Phosphonat ist, wobei A und C über eine Brücke von 3 - 8 Methylengruppen (B) voneinander getrennt sind. Aus dieser Verbindungsklasse zeigten Arylalkylphosphonsäuren mit Methylenbrücken von mehr als 5 Kohlenstoffatomen antivirale Aktivität gegen Herpes-Viren.

Arylalkylphosphonsäuren mit Methylenbrücken von weniger als 5 Kohlenstoffatomen Zeigen jedoch keine antivirale Aktivität. Die Substitution des aromatischen Restes dieser Verbindungen erfolgt bei Diana et al. im wesentlichen durch eine 2-Chlor-, 4-Methoxy- oder 4-Carbethoxyphenoxygruppe.

Benzylphosphonsäuren sind bisher nicht als wirksame antivirale Verbindungen beschrieben worden (J.C.H. Mao et al., Antimicrob. Agents Chemother. 27, 1985, 197).

Überraschend wurde nun gefunden, daß 2-Formylbenzylphosphonsäure-Derivate antivirale Aktivität aufweisen.

Die Erfindung betrifft daher eine Verbindung der Formel I,

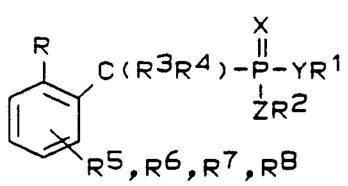

I

in der

R für eine Aldehydgruppe oder eine in einen Aldehyd überführbare Gruppe steht,

$R^1$ und $R^2$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium stehen oder $R^1$ und $R^2$ zusammen einen cyclischen Diester mit 2 bis 6 Kohlenstoffatomen im Ring bilden,

$R^3$ und $R^4$, die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom oder Jod stehen,

$R^5$, $R^6$, $R^7$ und $R^8$, die gleich oder verschieden sein können, für eine geradkettige oder verzweigte

Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom, Jod, eine Cyanid-, Hydroxy- oder Phenylgruppe stehen oder für den Rest der Formel Ia,

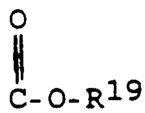

Ia

$R^{19}$ für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium steht und
X, Y und Z, die gleich oder verschieden sein können, für Sauerstoff oder Schwefel stehen
oder eine Prodrug-Form der Verbindung der Formel I.

Eine Verbindung der Formel I, in der
$R^1$ und $R^2$ für eine Alkylgruppe mit 1 bis 10 Kohlenstoffatomen, eine Alkenyl- oder Alkinylgruppe mit 2 bis 10 Kohlenstoffatomen, Wasserstoff oder eine Aralkylgruppe mit 7 bis 16 Kohlenstoffatomen stehen,
$R^3$ und $R^4$ für eine Alkylgruppe mit 1 bis 4 Kohlenstoffatomen, eine Alkenyl- oder Alkinylgruppe mit 2 bis 4 Kohlenstoffatomen oder Wasserstoff stehen,
$R^5$, $R^6$, $R^7$ und $R^8$ für Chlor, Brom, Methoxy oder Wasserstoff stehen und
X, Y und Z für Sauerstoff stehen,
ist bevorzugt.

Mit dem Begriff "Prodrug-Form der Verbindung der Formel I" sind Verbindungen gemeint, die auf dem Weg zum Wirkort in eine Verbindung der Formel I, in der R für eine Aldehydgruppe steht, umgewandelt werden. In dem Artikel von H. Bundgaard (Design of Prodrugs, 1985, S. 1 - 92, Elsevier-Verlag) wird der Begriff "Prodrug-Form" definiert und an Beispielen erläutert.

Unter der Bezeichnung Alkylgruppe mit 1 bis 10 Kohlenstoffatomen sind beispielsweise folgende Reste zu verstehen: Methyl, Ethyl, Propyl, Isopropyl, n-Butyl, sec.-Butyl, tert.-Butyl, 2,2-Dimethyl-1-propyl, n-Pentyl, n-Hexyl, n-Heptyl, n-Octyl, n-Nonyl und n-Decyl. Mit der Bezeichnung Alkenylgruppe mit 2 bis 10 Kohlenstoffatomen sind z. B. folgende Verbindungen gemeint: Ethenyl, Propenyl, Butenyl, Pentenyl, Hexenyl, Heptenyl, Octenyl, Nonenyl oder Decenyl. Mit der Bezeichnung Alkinylgruppe mit 2 bis 10 Kohlenstoffatomen sind z. B. folgende Verbindungen gemeint: Ethinyl, Propinyl, Butinyl, Pentinyl, Hexinyl, Heptinyl, Noninyl, Octinyl oder Decinyl. Unter einer Aralkylgruppe mit 7 bis 16 Kohlenstoffatomen sind z.B. folgende Reste zu verstehen:
Phenylmethyl, Phenylethyl, Phenylbutyl, Phenylpropyl, Phenylpentyl, Phenylhexyl, Phenylheptyl, Phenyloctyl, Phenylnonyl oder Phenyldecyl. Unter einer Cycloalkylgruppe mit 3 bis 8 Kohlenstoffatomen sind Reste zu verstehen wie Cyclopropyl, Cyclobutyl, Cyclopentyl, Cyclohexyl, Cycloheptyl oder Cyclooctyl. Alkoxygruppen mit 1 bis 4 Kohlenstoffatomen sind Reste wie Methoxy, Ethoxy, Propoxy, Isopropoxy, n-Butoxy, sec.-Butoxy oder tert.-Butoxy.

Die Erfindung betrifft ferner ein Verfahren zur Herstellung der Verbindung der Formel I, in der R für eine Aldehydgruppe steht, das dadurch gekennzeichnet ist, daß die Verbindung der Formel II,

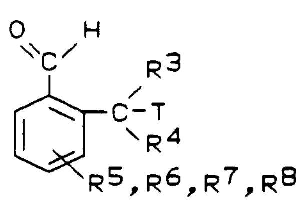

II

in der
$R^3$ und $R^4$, die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom oder Jod stehen,

$R^5$, $R^6$, $R^7$ und $R^8$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom, Jod, eine Cyanid-, Hydroxy- oder Phenylgruppe stehen oder für den Rest der Formel I a,

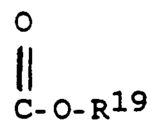

Ia

$R^{19}$ für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium steht und
T für Chlor, Brom, Jod, Methylsulfonat, Phenylsulfonat oder Tosylsulfonat steht,
mit der Verbindung der Formel III,

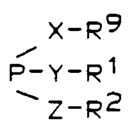

III

in der
$R^1$ und $R^2$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium stehen oder $R^1$ und $R^2$ zusammen einen cyclischen Diester mit 2 bis 6 Kohlenstoffatomen im Ring bilden,
$R^9$ für eine geradkettige oder verzweigte Alkylgruppe mit 1 bis 4 Kohlenstoffatomen steht und
X, Y und Z, die gleich oder verschieden sein können, für Sauerstoff oder Schwefel stehen,
zur Reaktion gebracht wird.

Die Erfindung betrifft ferner ein Verfahren zur Herstellung der Verbindung der Formel I, in der R für eine in einen Aldehyd überführbare Gruppe steht, das dadurch gekennzeichnet ist, daß die Verbindung der Formel I, in der R für Aldehyd steht, so umgesetzt wird, daß eine in einen Aldehyd überführbare Gruppe entsteht.

Unter dem Begriff "in einen Aldehyd überführbare Gruppe" werden Reste verstanden, die auf dem Weg zum Wirkort in einen Aldehyd umgewandelt werden (H. Bundgaard, Design of Prodrugs, 1985, S. 1 - 92, Elsevier-Verlag).

Insbesondere kann die Aldehydgruppe so derivatisiert werden, daß die Verbindung der Formel I entsteht, in der R für eine in einen Aldehyd überführbare Gruppe der Formel Ib, Ic oder Id steht,

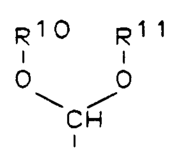

Ib

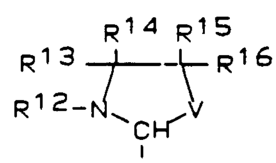

Ic

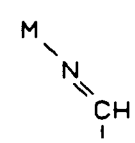

Id

in der $R^{10}$ und $R^{11}$, die gleich oder verschieden sein können, für eine geradkettige oder verzweigte Alkylgruppe mit 1 bis 10 Kohlenstoffatomen stehen oder $R^{10}$ und $R^{11}$ zusammen ein cyclisches Acetal mit 2

oder 3 Kohlenstoffatomen im Ring bilden,
$R^{12}$ bis $R^{16}$, die gleich oder verschieden sein können, für eine geradkettige oder verzweigte Alkylgruppe mit 1 bis 10 Kohlenstoffatomen oder eine Arylgruppe mit 6, 10 oder 14 Kohlenstoffatomen stehen,
V für Sauerstoff oder Schwefel steht,
M für eine Hydroxygruppe, eine geradkettige oder verzweigte Alkylgruppe mit 1 bis 10 Kohlenstoffatomen, eine Aralkylgruppe mit 7 bis 20 Kohlenstoffatomen, eine Arylgruppe mit 6, 10 oder 14 Kohlenstoffatomen steht oder für einen Rest der Formel Ie oder If,

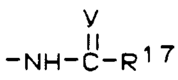

Ie

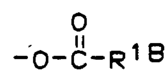

If

in der $R^{17}$ für eine geradkettige oder verzweigte Alkylgruppe mit 1 bis 10 Kohlenstoffatomen, eine Amino-, Pyridin- oder eine Arylgruppe mit 6, 10 oder 14 Kohlenstoffatomen steht und
$R^{18}$ für eine Aminogruppe, eine Pyridingruppe, eine geradkettige oder verzweigte Alkylgruppe mit 1 bis 10 Kohlenstoffatomen, eine Arylgruppe mit 6, 10 oder 14 Kohlenstoffatomen oder eine Aralkylgruppe mit 7 bis 20 Kohlenstoffatomen steht.

Die Synthese der Verbindung der Formel I, in der R für eine Aldehydgruppe steht, erfolgt durch Umsetzung der Verbindung der Formel II mit der Verbindung der Formel III zweckmäßig bei Temperaturen zwischen 100 und 250 °C, bevorzugt zwischen 120 und 180 °C (US 4 299 615; Houben-Weyl, Methoden der Org. Chemie, Vol. XII/1, Seite 423, Thieme-Verlag, Stuttgart; Houben-Weyl, Methoden der Org. Chemie, Vol. E2, Seite 300). Die Umsetzung kann in einem geeigneten Lösungsmittel, wie Hexamethylphosphorsäureamid (HMPA), Dimethylylformamid (DMF), Dimethylsulfoxid (DMSO), N,N'-Dimethyl-N,N'-propylenharnstoff (DMPU) oder N,N'-Dimethyl-N,N'-ethylenharnstoff (DMEU) erfolgen. Die Reaktion kann auch ohne Lösungsmittel durchgeführt werden. Die Reinigung erfolgt nach allgemein üblichen Methoden, bevorzugt durch Chromatographie über Kieselgel mit geeigneten Laufmitteln, durch Destillation oder durch Umkristallisation aus geeigneten Lösungsmitteln.

Die Verbindungen der Formel II und Formel III können in an sich bekannter Weise dargestellt werden. Die Überführung der Phosphonsäurediester in ihre Monoester, sowie in die entsprechenden freien Säuren oder deren Salze erfolgt beispielsweise durch Kochen mit verdünnter Salzsäure (Houben-Weyl, Methoden der Organischen Chemie, Vol. XII/1, 1963), oder durch Umsetzung mit Trimethylbromsilan (C.E. McKenna, J. Schmidhauser, J.C.S. Chem. Commun., 1979, 739). Die Reinigung erfolgt durch Umkristallisation in geeigneten Lösungsmitteln oder durch chromatographische Methoden, bevorzugt durch Ionenaustauschchromatographie mit geeigneten Laufmitteln. Durch Ionenaustauschchromatographie können auch die gewünschten Salzformen erhalten werden.

Die Synthese einer Prodrug-Form der Verbindung der Formel I kann beispielsweise dadurch erfolgen, daß die Aldehydgruppe in der Verbindung der Formel I so derivatisiert wird, daß Verbindungen wie Oxime, Thiosemicarbazone, Carbonsäurehydrazone, Schiffsche Basen, Oxazolidine, Thiazolidine oder Acetale enstehen. Dazu kann die Verbindung der Formel I, in der R für eine Aldehydgruppe steht, mit der Verbindung der Formel IVa, IVb und/oder IVc, IVd oder IVe,

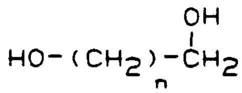

IVa

$R^{10}-OH$

IVb

$R^{11}-OH$

IVc

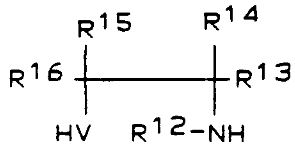

IVd

$M-NH_2$

IVe

in der $R^{10}$ bis $R^{16}$, M und V die genannte Bedeutung haben und n für 1 oder 2 steht, zur Reaktion gebracht werden.

Weitere Prodrug-Formen ergeben sich in analoger Weise aus den in Bundgaard beschriebenen Methoden.

Die an der Aldehydgruppe derivatisierten Verbindungen der Formel I können in vitro und in vivo in die aktive, antiviral wirksame Form (Aldehyd-Form) überführt werden (H. Bundgaard, Design of Prodrugs, 1985, 1 - 92, Elsevier-Verlag). Die Überführung in die aktive Form kann z. B. durch Hydrolyse in wäßriger Lösung oder durch enzymatische Katalyse am oder auf dem Weg zum Wirkort geschehen.

Ein Wirksamkeitstest von Chemotherapeutika für HIV-Infektionen beim Menschen bereitet Schwierigkeiten, da noch kein Infektionsmodell bei Labortieren existiert. Für die Prüfung von Chemotherapeutika muß deshalb auf die Infektion mit anderen Retroviren zurückgegriffen werden. In diesem Fall wurde die Infektion der Maus mit dem Friend-Leukämie-Virus gewählt. Dazu wurden normale NMRI-Labormäuse (NMRI = Naval Medical Research Institute) durch intravenöse Injektion mit Friend-Leukämie-Virus enthaltendem Mäuseserum infiziert. Bei den unbehandelten Kontrolltieren entwickelte sich als Symptom der Infektion innerhalb von 2 Wochen eine deutliche Vergrößerung von Milz und Leber. Die Behandlung erfolgte über 10 Tage, beginnend 48 Stunden nach der Infektion. Am 14. Versuchstag wurden die Tiere getötet und geöffnet. Die Milz wurde entnommen und gewogen. Als Meßparameter der therapeutischen Wirksamkeit wurde das Milzgewicht der behandelten Tiere mit dem der unbehandelten Infektionskontrolle in Relation gesetzt.

Bei uninfizierten ausgewachsenen Labormäusen (20 - 24 g Körpergewicht) wog die Milz etwa 1 % des Körpergewichts oder weniger, während bei infizierten Tieren die Milz am Ende des Versuchs etwa 10 % des Körpergewichts erreichte.

Die Verbindung der Formel I, in der R für eine Aldehydgruppe steht, besitzt wertvolle pharmakologische Eigenschaften, insbesondere eine antivirale Wirkung und zwar gegen sowohl durch DNA- als auch durch RNA-Viren verursachte Krankheiten, besonders gegen Krankheiten, die hervorgerufen werden durch Herpes simplex Virus (HSV I), Myxoviren, Friend-Leukämie-Virus (FLV) oder Human Immunodeficiency Virus (HIV). Die erfindungsgemäßen Verbindungen eignen sich daher zur Bekämpfung verschiedener durch Viren verursachter Krankheiten, wie der Erkrankung des Respirationstraktes, Erkrankungen der Haut, des Auges, des zentralen Nervensystems, AIDS und AIDS verwandter Zustände, wie des AIDS verwandten Komplexen (ARC), generalisierte Lymphadenopathie (PGL), AIDS-verwandten, neuralgischen Zuständen (wie Schwachsinn oder tropische Paraperese), Anti-HIV-Antikörper-positive Zustände, Kaposi-Sarkom oder thrombopenische Purpura.

Die Verbindung der Formel I und/oder ihre Prodrug-Form können entweder allein oder mit physiologisch verträglichen Hilfs- oder Trägerstoffen vermischt in wirksamen Mengen als Arzneimittel angewandt werden. Sie kann beispielsweise oral in einer Dosis von 1 bis 500 mg/kg/Tag, vorzugsweise 5 bis 50 mg/kg/Tag verabreicht werden. Die Applikation für parenterale, rectale oder topische Anwendung oder als Aerosol erfolgt z. B. in einer Menge von 0,5 bis 500 mg/kg/Tag, bevorzugt mit 2 bis 100 mg/kg/Tag. Die Verbindung der Formel I und/oder ihre Prodrug-Form werden zweckmäßig in Dosierungseinheiten verabreicht, die mindestens die wirksame Menge der erfindungsgemäßen Verbindungen, bevorzugt 25 bis 6.000

mg, besonders bevorzugt 100 bis 1.000 mg enthalten. Diese Werte beziehen sich auf einen erwachsenen Menschen mit einem Gewicht von 75 kg. Diese Dosierungseinheiten können auch mehrmals pro Tag verabreicht werden. Die Dosierung kann in schweren Fällen auch erhöht werden. In vielen Fällen genügen jedoch auch geringere Mengen. Zur Bekämpfung von Krankheiten, die durch RNA- oder DNA-Viren verursacht werden, eignen sich insbesondere
2-Formylbenzylphosphonsäure-diethylester,
2-Formylbenzylphosphonsäure-ditriethylammoniumsalz
2-Formylbenzylphosphonsäure-monoethylestertriethylammoniumsalz,
2-Formylbenzylphosphonsäurediethylester-thiosemicarbazon,
2-Formylbenzylphosphonsäurediethylester-nicotinsäurehydrazon
oder
2-(3,4-Dimethyl-5-phenyloxazolidin-2-yl)-benzylphosphonsäurediethylester.

Die erfindungsgemäße Verbindung der Formel I und/oder ihre Prodrug-Form kann auch in Kombination mit anderen Stoffen, insbesondere Antivirusmitteln und Immunstimulatoren, wie Interferonen verabreicht werden. Die Verbindung der Formel I und/oder ihre Prodrug-Form werden im folgenden als Wirkstoff bezeichnet.

Die Erfindung umfaßt weiterhin die Verwendung des Wirkstoffes bei der Herstellung von Arzneimitteln, die zur Behandlung und Prophylaxe der vorstehend genannten Krankheiten eingesetzt werden. Ein weiterer Gegenstand der Erfindung sind Arzneimittel, die einen oder mehrere Wirkstoffe enthalten.

Die Arzneimittel werden nach an sich bekannten, dem Fachmann geläufigen Verfahren hergestellt. Als Arzneimittel wird der Wirkstoff entweder als solcher oder vorzugsweise in Kombination mit geeigneten pharmazeutischen Hilfs- oder Trägerstoffen in Form von Tabletten, Dragees, Kapseln, Suppositorien, Emulsionen, Suspensionen oder Lösungen eingesetzt, wobei der Wirkstoffgehalt bis etwa 95 %, vorteilhafterweise zwischen 10 und 75 %, beträgt.

Geeignete Hilfs- bzw. Trägerstoffe für die gewünschte Arzneimittelformulierung sind beispielsweise neben Lösemitteln, Gelbildnern, Suppositoriengrundlagen, Tabletten-Hilfsstoffen und anderen Wirkstoffträgern auch Antioxidantien, Dispergiermittel, Emulgatoren, Entschäumer, Geschmackskorrigentien, Konservierungsmittel, Lösungsvermittler oder Farbstoffe.

Der Wirkstoff kann oral, parenteral, intravenös oder rectal appliziert werden, wobei neben der oralen Applikation insbesondere die intranasale Applikation als Aerosol bevorzugt ist.

Für eine orale Anwendungsform wird der Wirkstoff mit den dafür geeigneten Zusatzstoffen wie Trägerstoffen, Stabilisatoren oder inerten Verdünnungsmitteln vermischt und durch die üblichen Methoden in eine geeignete Darreichungsform gebracht, wie Tabletten, Dragees, Steckkapseln, wäßrige oder ölige Lösungen. Als inerte Trägerstoffe können z. B. Gummi arabicum, Magnesia, Magnesiumcarbonat, Kaliumphosphat, Milchzucker, Glukose oder Stärke, insbesondere Maisstärke verwendet werden. Dabei kann die Zubereitung sowohl als Trocken- als auch als Feuchtgranulat erfolgen. Als ölige Trägerstoffe oder Lösemittel kommen beispielsweise pflanzliche oder tierische Öle in Betracht, wie Sonnenblumenöl oder Lebertran.

Zur subkutanen oder intravenösen Applikation wird der Wirkstoff mit den dafür geeigneten Substanzen wie Lösungsvermittler, Emulgatoren oder weiteren Hilfsstoffen in Lösung, Suspension oder Emulsion gebracht. Als Lösungsmittel kommen z. B. physiologische Kochsalzlösung, Alkohole, z. B. Ethanol, Propanol, Glycerin, Zuckerlösungen wie Glucose- oder Mannitlösungen oder eine Mischung von Lösungsmitteln in Frage.

Die nachfolgenden Beispiele dienen der weiteren Erläuterung der Erfindung.

**Beispiel 1**

Herstellung von 2-Formylbenzylphosphonsäure-diethylester (A)

47,9 g (0,31 mol) 2-Chlormethylbenzaldehyd wurden zusammen mit 51,5 g (0,31 mol) Triethylphosphit auf 160 °C erhitzt. Dabei destillierte Ethylchlorid ab. Das Produkt wurde durch fraktionierte Destillation gereinigt.
Ausbeute: 64,5 9 (81 %); Sdp.: 130 °C/0,3 mm;
1H-NMR (270 MHz, $CDCl_3$/TMS): $\delta$ = 1,23 (t,6H, P-O-$CH_2$-$CH_3$), 3,78 (d,2H,$CH_2$-P) JP-H = 24 Hz, 4,04 (dq,4H, P-O-$CH_2$-$CH_3$), 7,19 - 7,97 (m,4H,Ar-H)

**Beispiel 2**

Herstellung von 2-Formylbenzylphosphonsäure-ditriethylammoniumsalz(B) und 2-Formylbenzylphosphonsäure-monoethylester-triethylammoniumsalz (C)

5,0 g (20 mmol) 2-Formylbenzylphosphonsäure-diethylester wurden mit 100 ml 6 M HCl versetzt und 6 h unter Rückfluß gekocht. Wasser und HCl wurden im Vakuum abdestilliert, es wurde dreimal mit Toluol koevaporiert. Die zurückgebliebene braune Masse wurde über Kieselgel ($CH_2Cl_2$/Methanol/Triethylamin 75/24/1) chromatographiert. Verbindungen B und C wurden als ölige Produkte erhalten. Sie konnten durch Chromatographie über Diethylaminoethyl-®Sephadex A25 ($Et_3NH^+$ Form, Firma Pharmacia, Freiburg, BRD) getrennt werden. Sie unterscheiden sich in ihrem Laufverhalten (Rf-Wert). Die Elution erfolgte mit einem Triethylammoniumbicarbonat-Gradienten von 0,3 - 1,0 M.

(9): Rf = 0,1; Ausbeute: 2,4 g (30 %); 1HNHR (270 MHz, DMSO/TMS): $\delta$ = 1,07 (t,18H,N-$CH_2$-$CH_3$), 2,86 (g,12H,N-$CH_2$-$CH_3$), 3,23 (d,2H,$CH_2$-P) JP-H = 23 Hz, 7,24 - 7,78 (m, 4H,Ar-H), 10,31 (s,1H,CHO).

(C): Rf = 0,3; Ausbeute: 1,4 g (21 %); 1HNMR (270 MHz, DM50/TMS): $\delta$ = 1,01 - 1,17 (m,12H, N-$CH_2$-$CH_3$ & P-O-$CH_2$-$CH_3$), 2,88 (g,6H,N-$CH_2$-$CH_3$), 3,27 ((d,2H,$CH_2$-P) JP-H 23 Hz, 3,68 ((dq,2H, P-O-$CH_2$-$CH_3$), 7,18 - 7,78 (m,4H,Ar-H), 10,31 (s,1H,CHO).

**Beispiel 3**

Herstellung von 2-Formylbenzylphosphonsäureditriethylammoniumsalz (B)

Zu 2,0 g (8 mmol) der Verbindung A in 10 ml absoluten Dioxan wurden 3,2 g (21 mmol) Trimethylsilylbromid getropft, das Reaktionsgemisch wurde auf 50 °C erhitzt und 6 h bei dieser Temperatur gerührt. Es wurde evaporiert, mehrfach mit Wasser versetzt und lyophilisiert. Das Rohprodukt wurde durch Chromatographie wie in Beispiel 2 gereinigt.
Ausbeute: 1,98 g (62 %).

**Beispiel 4**

2-Formylbenzylphosphonsäurediethylester-thiosemicarbazon (D)

2,0 g (8 mmol) der Verbindung A und 0,73 g Thiosemicarbazid wurden in 200 ml absoluten Ethanol gelöst bzw. suspendiert. Es wurden 2 ml Essigsäure zugegeben und 3 h unter Rückfluß gekocht. Während des langsamen Abkühlens fiel das Produkt D kristallin aus.

Ausbeute: 1,8 g (68 %); Fp.: 195 bis 197 °C; $^1$HNMR (270 MHz, $CDCl_3$/TMS): $\delta$ = 1,16 (t,6H,$CH_2$-$CH_3$), 3,38 (d,2H,$CH_2$-P, $J_{P-H}$ = 23 Hz), 3,94 (dq,4H, $CH_2$-$CH_3$), 7,23 - 7,39 (m,3H,Ar-H), 8,40 (s,1H,Ar-H), 11,37 (s,1H,Ar-CH = N).

**Beispiel 5**

2-Formylbenzylphosphonsäurediethylester-nicotinsäurehydrazon (E)

2,0 g (8 mmol) der Verbindung A und 1,07 g (8 mmol) Nicotinsäurehydrazid wurden in 30 ml absoluten Ethanol gelöst. Nach Zugabe von 1 ml Essigsäure wurde 8 h unter Rückfluß gekocht. Das Lösungsmittel wurde einrotiert, der Rückstand über Kieselgel chromatographiert (Laufmittel $CH_2Cl_2$/EtOH 9,5/0,5; Rf = 0,45). Das Produkt E wurde kristallin erhalten.

Ausbeute: 2,2 g (73 %); Fp.: 136 bis 140 °C; $^1$H-NMR (270 MHz, $CDCl_3$/TMS): $\delta$ = 1,14 - 1,37 (m,6H,$CH_3$), 3,61 - 3,79 (d,2H,$CH_2$-P), $J_{P-H}$ = 24 Hz, 3,87 - 4,16 (m,4H,$CH_2$-$CH_3$), 7,11 - 7,49 (m,4H,Ar-H), 7,70 - 9,23 (m,5H,Py-H), 10,17 & 11,25 (je s, Vh. 1 : 3, 1H,NH).

**Beispiel 6**

2-(3,4-Dimethyl-5-phenyloxazolidin-2-yl)-benzylphosphonsäure-diethylester (F)

2,0 g (8 mmol) der Verbindung A und 1,32 g (8 mmol) (-)-Ephedrin wurden in 100 ml Benzol gelöst und 24 h am Wasserabscheider unter Rückfluß gekocht. Danach wurde das Lösungsmittel einrotiert, der Rückstand über Kieselgel chromatographiert (Laufmittel $CH_2Cl_2$/Ethanol 9,5/0,5; Rf = 0,55). Das Produkt F

wurde als Öl erhalten.

Ausbeute: 2,4 g (75 %); $^1$H-NMR (270 MHz, $CDCl_3$/TMS): $\delta$ = 0,80, (d,3H,CH-$CH_3$), 1,25 (m,6H,O-$CH_2$-$CH_3$), 2,27 (s,3H,N-$CH_3$), 3,18 (dq,1H,CH-$CH_3$), 3,20 & 3,28 (dd,1H, Ph-CH-CH), 3,59 - 3,76 (m,2H,$CH_2$-P); 4,01 (m,4H,O-$CH_2$-$CH_3$), 5,19 (s,1H,Ar-CH(O-)(N-), 7,16 - 7,45 (m,8H,Ar-H), 7,89 - 7,98 (m,1H,Ar-H).

**Beispiel 7**

Pathogenfreie NMRI-Mäuse mit einem Gewicht von etwa 15 g wurden intraperitoneal mit Herpes simplex Typ 1 infiziert und anschließend mit den in Tabelle 1 genannten Verbindungen intraperitoneal, oral oder subkutan therapiert. Die Behandlung erfolgte zweimal täglich über 2,5 Tage, beginnend nach der Infektion. Der Behandlungserfolg wurde anhand des Krankheitsverlaufes und der Überlebensrate gegenüber den unbehandelten Infektionskontrollen bestimmt. Die Kontrollen erhielten anstelle der zu prüfenden Verbindungen eine wasserlösliche Methylhydroxyethylcellulose (Viskosität 300 Pa•s in 2 %iger Lösung appliziert). Die Versuche wurden mit Gruppen von je 5 Mäusen pro Präparat durchgeführt.

Die chemotherapeutische Wirkung der Verbindung A ist aus Tabelle 1 ersichtlich.

**Tabelle 1** Herpes simplex 1

| Präparat | Dosierung (mg/kg) | Überlebende | Mittlere Überlebenszeit (Tage) |
|---|---|---|---|
| Kontrolle | sc 0 | 1 | 6,7 |
| A | sc 2,5 | 2 | 9,0 |
| A | sc 25 | 0 | 7,2 |
| A | sc 250 | 0 | 8,4 |
| Kontrolle | po 0 | 1 | 8,0 |
| A | po 2,5 | 4 | 8,0 |
| A | po 25 | 5 | --- |
| A | po 250 | 4 | 8,0 |
| Kontrolle | sc 0 | 1 | 8,3 |
| C | sc 3 | 1 | 8,5 |
| C | sc 10 | 3 | 8,0 |
| C | sc 30 | 4 | 10,0 |
| Kontrolle | po 0 | 1 | 7,8 |
| C | po 3 | 4 | 9,0 |
| C | po 10 | 3 | 8,0 |
| C | po 30 | 1 | 8,3 |
| Kontrolle | sc 0 | 1 | 8,3 |
| B | sc 3 | 2 | 7,8 |
| B | sc 10 | 3 | 7,0 |
| B | sc 30 | 3 | 6,5 |

**Tabelle 1, Forts.** Herpes simplex 1

| Präparat | Dosierung (mg,/kg) | Überlebende | Mittlere Überlebenszeit (Tage) |
|---|---|---|---|
| Kontrolle | po 0 | 1 | 7,8 |
| B | po 3 | 3 | 7,0 |
| B | po 10 | 2 | 8,3 |
| B | po 30 | 3 | 6,0 |
| Kontrolle | ip 0 | 1 | 8,3 |
| A | ip 3 | 0 | 8,0 |
| A | ip 10 | 4 | 9,0 |
| A | ip 30 | 3 | 8,5 |

po = oral
sc = subkutan
ip = intraperitoneal

**Beispiel 8**

Zellkulturen von Hela- und Vero-Zellen wurden in Mikrotiterplatten überimpft und mit Myxoviren (Influenza A2) infiziert. 2 Stunden nach der Infektion wurden die Verbindungen B und C den infizierten Zellkulturen in verschiedenen Verdünnungen zugegeben. 48 bis 72 Stunden nach der Infektion wurde der Therapieerfolg anhand des cytopathogenen Effektes mikroskopisch und nach Neutralrotaufnahmen (Farbtest nach Finter) photometrisch bestimmt (Finter, N.B. Interferons, 1966). Die minimale Konzentration, bei der etwa die Hälfte der infizierten Zellen keinen cytopathogenen Effekt zeigen, wird als minimale Hemmkonzentration (MHK) betrachtet. Die Ergebnisse sind in der Tabelle 2 zusammengefaßt.

**Tabelle 2** Influenza A2

| Substanz | MHK (µg/ml) | DTM (µg/ml) |
|---|---|---|
| C | 44,4 | > 400 |
| B | 4,94 | > 400 |

MHK = Minimale Hemmkonzentration
DTM = Dosis tolerata maxima

**Beispiel 9**

Pathogenfreie NMRI-Mäuse mit einem Gewicht von etwa 16 g wurden intranasal mit Influenza A2 infiziert und anschließend mit den in Tabelle 3 genannten Verbindungen subkutan und oral therapiert. Die Verbindungen wurden den Tieren unter leichter Ethernarkose mit je einem Tropfen Virussuspension in Nasenlöcher appliziert. Die Behandlung erfolgte zweimal täglich über 2,5 Tage, beginnend nach der Infektion. Als Vergleich diente stets Amantadin. Der Behandlungserfolg wurde anhand des Krankheitsverlaufs und der Überlebensrate gegenüber den unbehandelten Infektionskontrollen bestimmt. Die Kontrollen erhielten anstelle der zu prüfenden Verbindungen eine wasserlösliche Methylhydroxyethylcellulose (Viskosität 300 Pa•s in 2 %iger Lösung) appliziert. Die Versuche wurden mit Gruppen von je 5 Mäusen pro Präparat durchgeführt.

Die chemotherapeutische Wirkung ergibt sich aus Tabelle 3.

**Tabelle 3** Influenza A2

| Präparat | Dosierung (mg/kg) | Anzahl der Überlebenden | Mittlere Überlebenszeit (Tage) |
|---|---|---|---|
| Kontrolle | po 0 | 2 | 7,5 |
| Amantadin | po 80 | 5 | --- |
| A | po 2,5 | 4 | 9,0 |
| A | po 3 | 4 | 8,0 |
| A | po 10 | 4 | 7,0 |
| A | po 25 | 2 | 6,3 |
| A | po 30 | 2 | 7,3 |
| Kontrolle | sc 0 | 0 | 6,6 |
| Amantadin | sc 80 | 5 | --- |
| A | sc 2,5 | 2 | 6,7 |
| A | sc 25 | 3 | 7,0 |
| Kontrolle | sc 0 | 2 | 6,7 |
| Amantadin | sc 80 | 5 | --- |
| B | sc 1,5 | 1 | 8,5 |
| B | sc 15 | 4 | 8,0 |

**Tabelle 3, Forts.** Influenza A2

| Präparat | Dosierung (mg/kg) | Anzahl der Überlebenden | Mittlere Überlebenszeit (Tage) |
|---|---|---|---|
| Kontrolle | po 0 | 1 | 6,5 |
| Amantadin | po 80 | 4 | 8,0 |
| B | po 1,5 | 3 | 8,5 |
| B | po 15 | 5 | --- |
| B | po 150 | 3 | 7,0 |
| Kontrolle | sc 0 | 0 | 6,8 |
| C | sc 0,25 | 5 | --- |
| C | sc 2,5 | 5 | --- |
| C | sc 25 | 3 | 7,5 |
| Kontrolle | po 0 | 0 | 7,2 |
| C | po 0,25 | 5 | --- |
| C | po 2,5 | 2 | 6,0 |
| C | po 25 | 5 | --- |
| Kontrolle | po 0 | 0 | 6,6 |
| D | po 0,25 | 4 | 8,0 |
| D | po 2,5 | 1 | 7,0 |
| D | po 25 | 2 | 7,3 |
| Kontrolle | po 0 | 0 | 6,6 |
| E | po 0,25 | 4 | 8,0 |
| E | po 2,5 | 3 | 6,7 |
| E | po 25 | 3 | 6,0 |
| Kontrolle | po 0 | 1 | 7,0 |
| F | po 0,25 | 3 | 6,5 |
| F | po 2,5 | 3 | 6,5 |
| F | po 25 | 2 | 7,0 |

sc = subkutan
po = oral

**Beispiel 10**

Labormäuse (NMRI, weiblich, Gewicht 20 - 24 g) wurden mit Friend-Leukämie-Virus (FLV)-haltigem Mäuseserum intravenös infiziert. 48 h nach Infektion wurde mit der Behandlung begonnen. Über 10 Tage wurden die Mäuse mit den in Tabelle 4 angegebenen Substanzen behandelt. Einmal pro Tag wurden die angegebenen Substanzen oral oder intraperitoneal verabreicht. 14 Tage nach Infektion wurden die Tiere durch Luxation getötet und die Milzen wurden entnommen. Das Gewicht der Milzen wurde bestimmt. Als Meßparameter der therapeutischen Wirksamkeit wurde das Milzgewicht der Tiere, die mit Verbindung A und D behandelt wurden, mit dem der unbehandelten Infektionskontrolle in Relation gesetzt.

Als Standardsubstanz diente Suramin und Azidothymidin (AZT). Die Wirkung der Präparate ergibt sich aus Tabelle 4.

**Tabelle 4** Friend-Leukämie-Virus

| Präparat | Dosierung | Überlebens-rate % | relatives Milzgewicht % Körper-gewicht |
|---|---|---|---|
| Kontrolle | po 0 | 100 | 12,36 |
| AZT | po 15,5 | 100 | 3,26 |
| A | po 17,5 | 100 | 7,74 |
| A | po 81 | 100 | 5,67 |
| Kontrolle | ip 0 | 100 | 10,64 |
| AZT | ip 50,0 | 100 | 7,60 |
| D | ip 50,0 | 100 | 7,60 |
| Kontrolle | ip 0 | 100 | 12,36 |
| Suramin | ip 50 | 70 | 6,06 |
| A | ip 25 | 90 | 6,27 |
| A | ip 50 | 100 | 6,52 |

ip = intraperitoneal
po = oral

**Patentansprüche**
**Patentansprüche für folgende Vertragsstaaten : AT, BE, CH, DE, DK, FR, GB, IT, LI, LU, NL, SE**

1. Verbindung der Formel I,

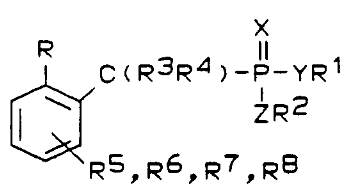

I

in der
R für eine Aldehydgruppe oder eine in einen Aldehyd überführbare Gruppe steht,
$R^1$ und $R^2$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium stehen oder $R^1$ und $R^2$ zusammen einen cyclischen Diester mit 2 bis 6 Kohlenstoffatomen im Ring bilden,
$R^3$ und $R^4$, die gleich oder verschieden sein können, für eine geradkettige oder verzweigte Alkylgruppe

mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom oder Jod stehen,

$R^5$, $R^6$, $R^7$ und $R^8$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom, Jod, eine Cyanid-, Hydroxy- oder Phenylgruppe stehen oder für den Rest der Formel Ia,

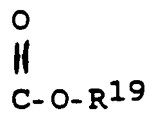

Ia

$R^{19}$ für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium steht und

X, Y und Z, die gleich oder verschieden sein können, für Sauerstoff oder Schwefel stehen

oder eine Prodrug-Form der Verbindung der Formel I.

**2.** Verbindung der Formel I nach Anspruch 1, in der

$R^1$ und $R^2$ für eine Alkylgruppe mit 1 bis 10 Kohlenstoffatomen, eine Alkenyl- oder Alkinylgruppe mit 2 bis 10 Kohlenstoffatomen, Wasserstoff oder eine Aralkylgruppe mit 7 bis 16 Kohlenstoffatomen stehen,

$R^3$ und $R^4$ für eine Alkylgruppe mit 1 bis 4 Kohlenstoffatomen, eine Alkenyl- oder Alkinylgruppe mit 2 bis 4 Kohlenstoffatomen oder Wasserstoff stehen,

$R^5$, $R^6$, $R^7$ und $R^8$ für Chlor, Brom, Methoxy oder Wasserstoff stehen und

X, Y und Z für Sauerstoff stehen.

**3.** Verbindung nach Anspruch 1, in der R für eine in einen Aldehyd überführbare Gruppe der Formel Ib, Ic oder Id steht,

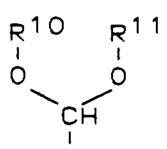

Ib

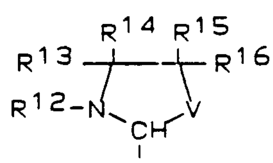

Ic

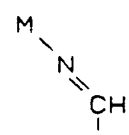

Id

in der

$R^{10}$ und $R^{11}$, die gleich oder verschieden sein können, für eine geradkettige oder verzweigte Alkylgruppe mit 1 bis 10 Kohlenstoffatomen stehen oder $R^{10}$ und $R^{11}$ zusammen ein cyclisches Acetal mit 2 oder 3 Kohlenstoffatomen im Ring bilden,

$R^{12}$ bis $R^{16}$, die gleich oder verschieden sein können, für eine geradkettige oder verzweigte Alkylgruppe mit 1 bis 10 Kohlenstoffatomen oder eine Arylgruppe mit 6, 10 oder 14 Kohlenstoffatomen stehen,

V für Sauerstoff oder Schwefel steht,

M für eine Hydroxygruppe, eine geradkettige oder verzweigte Alkylgruppe mit 1 bis 10 Kohlenstoffatomen, eine Aralkylgruppe mit 7 bis 20 Kohlenstoffatomen, eine Arylgruppe mit 6, 10 oder 14 Kohlenstoffatomen steht oder für einen Rest der Formel Ie oder If,

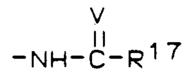

Ie

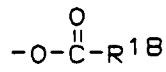

If

in der $R^{17}$ für eine geradkettige oder verzweigte Alkylgruppe mit 1 bis 10 Kohlenstoffatomen, eine Amino-, Pyridin- oder Arylgruppe mit 6, 10 oder 14 Kohlenstoffatomen steht und
$R^{18}$ für eine Aminogruppe, eine Pyridingruppe, eine geradkettige oder verzweigte Alkylgruppe mit 1 bis 10 Kohlenstoffatomen, eine Arylgruppe mit 6, 10 oder 14 Kohlenstoffatomen oder eine Aralkylgruppe mit 7 bis 20 Kohlenstoffatomen steht.

**4.** 2-Formylbenzylphosphonsäure-diethylester.

**5.** 2-Formylbenzylphosphonsäure-ditriethylammoniumsalz.

**6.** 2-Formylbenzylphosphonsäure-monoethylestertriethylammoniumsalz.

**7.** 2-Formylbenzylphosphonsäurediethylester-thiosemicarbazon.

**8.** Verfahren zur Herstellung der Verbindung der Formel I nach Anspruch 1, in der R für eine Aldehydgruppe steht, dadurch gekennzeichnet, daß die Verbindung der Formel II,

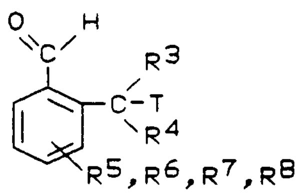

II

in der $R^3$ und $R^4$, die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom oder Jod stehen,
$R^5$, $R^6$, $R^7$ und $R^8$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom, Jod, eine Cyanid-, Hydroxy- oder Phenylgruppe stehen oder für den Rest der Formel Ia,

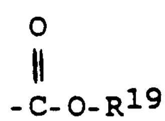

Ia

$R^{19}$ für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium steht und
T für Chlor, Brom, Jod, Methylsulfonat, Phenylsulfonat oder Tosylsulfonat steht,
mit der Verbindung der Formel III,

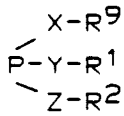

III

in der $R^1$ und $R^2$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium stehen oder $R^1$ und $R^2$ zusammen einen cyclischen Diester mit 2 bis 6 Kohlenstoffatomen im Ring bilden,
$R^9$ für eine geradkettige oder verzweigte Alkylgruppe mit 1 bis 4 Kohlenstoffatomen steht und
X, Y und Z, die gleich oder verschieden sein können, für Sauerstoff oder Schwefel stehen,
zur Reaktion gebracht werden.

**9.** Verfahren zur Herstellung der Verbindung der Formel I nach Anspruch 1, in der R für eine in einen Aldehyd überführbare Gruppe steht, dadurch gekennzeichnet, daß die Verbindung der Formel I, in der R für Aldehyd steht, so umgesetzt wird, daß eine in einen Aldehyd überführbare Gruppe entsteht.

**10.** Verfahren zur Herstellung der Verbindung der Formel I nach Anspruch 3, dadurch gekennzeichnet, daß die Verbindung der Formel I, in der R für eine Aldehydgruppe steht, mit der Verbindung der Formel IVa, IVb und/oder IVc, IVd oder IVe,

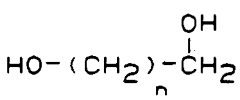

IVa

$R^{10}-OH$

IVb

$R^{11}-OH$

IVc

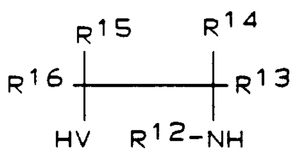

IVd

$M-NH_2$

IVe

in der $R^{10}$ und $R^{11}$ für eine geradkettige oder verzweigte Alkylgruppe mit 1 bis 10 Kohlenstoffatomen stehen,
$R^{12}$ bis $R^{16}$, die gleich oder verschieden sein können, für eine geradkettige oder verzweigte Alkylgruppe mit 1 bis 10 Kohlenstoffatomen oder eine Arylgruppe mit 6, 10 oder 14 Kohlenstoffatomen stehen,
V für Sauerstoff oder Schwefel steht,
M für eine Hydroxygruppe, eine geradkettige oder verzweigte Alkylgruppe mit 1 bis 10 Kohlenstoffatomen, eine Aralkylgruppe mit 7 bis 20 Kohlenstoffatomen, eine Arylgruppe mit 6, 10 oder 14 Kohlenstoffatomen steht oder für einen Rest der Formel Ie oder If,

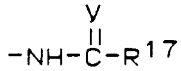

Ie

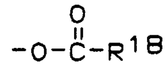

If

in der $R^{17}$ für eine geradkettige oder verzweigte Alkylgruppe mit 1 bis 10 Kohlenstoffatomen, eine

Amino-, Pyridin- oder eine Arylgruppe mit 6, 10 oder 14 Kohlenstoffatomen steht und
$R^{18}$ für eine Aminogruppe, eine Pyridingruppe, eine geradkettige oder verzweigte Alkylgruppe mit 1 bis 10 Kohlenstoffatomen, eine Arylgruppe mit 6, 10 oder 14 Kohlenstoffatomen oder eine Aralkylgruppe mit 7 bis 20 Kohlenstoffatomen steht,
zur Reaktion gebracht wird.

**11.** Verwendung der Verbindung der Formel I nach Anspruch 1 zur Herstellung eines Arzneimittels zur Behandlung von Erkrankungen, die durch RNA- oder DNA-Viren hervorgerufen werden.

**12.** Verwendung der Verbindung der Formel I nach Anspruch 2 zur Herstellung eines Arzneimittels zur Behandlung von Erkrankungen, die durch RNA- oder DNA-Viren hervorgerufen werden.

**13.** Verwendung der Verbindung der Formel I nach Anspruch 3 zur Herstellung eines Arzneimittels zur Behandlung von Erkrankungen, die durch RNA- oder DNA-Viren hervorgerufen werden.

**14.** Pharmazeutische Präparate enthaltend eine wirksame Menge der Verbindung nach Anspruch 1.

**15.** Verfahren zur Herstellung eines Arzneimittels, dadurch gekennzeichnet, daß eine wirksame Menge einer Verbindung nach Anspruch 1 in eine geeignete Darreichungsform überführt wird.

**Patentansprüche für folgende Vertragsstaaten : ES, GR**

**1.** Verfahren zur Herstellung der Verbindung der Formel I,

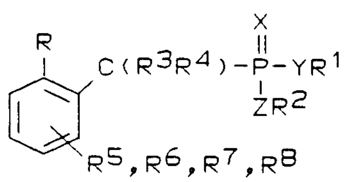

I

in der
R für eine Aldehydgruppe steht,
$R^1$ und $R^2$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium stehen oder $R^1$ und $R^2$ zusammen einen cyclischen Diester mit 2 bis 6 Kohlenstoffatomen im Ring bilden,
$R^3$ und $R^4$, die gleich oder verschieden sein können, für eine geradkettige oder verzweigte Alkylgruppe mit 1 bis 20 Kohlenstoffatomen, eine geradkettige oder verzweigte Alkinyl- oder Alkenylgruppe mit 2 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom oder Jod stehen,
$R^5$, $R^6$, $R^7$ und $R^8$, die gleich oder verschieden sein können, für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, eine Alkoxygruppe mit 1 bis 4 Kohlenstoffatomen, Wasserstoff, Fluor, Chlor, Brom, Jod, eine Cyanid-, Hydroxy- oder Phenylgruppe stehen oder für den Rest der Formel Ia,

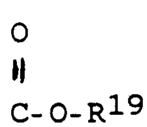

Ia

$R^{19}$ für eine geradkettige oder verzweigte Alkylgruppe mit 1 bis 20 Kohlenstoffatomen, eine geradkettige oder verzweigte Alkenyl- oder Alkinylgruppe mit 2 bis 20 Kohlenstoffatomen, eine Aralkylgruppe mit 7 bis 20 Kohlenstoffatomen, eine Cycloalkylgruppe mit 3 bis 8 Kohlenstoffatomen, Wasserstoff, Natrium, Kalium, Calcium, Magnesium, Aluminium, Lithium, Ammonium oder Triethylammonium steht und

X, Y und Z, die gleich oder verschieden sein können, für Sauerstoff oder Schwefel stehen dadurch gekennzeichnet, daß die Verbindung der Formel II,

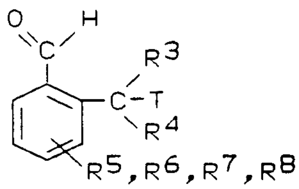

II

in der $R^3$, $R^4$, $R^5$, $R^6$, $R^7$ und $R^8$ die obengenannte Bedeutung haben und
T für Chlor, Brom, Jod, Methylsulfonat, Phenylsulfonat oder Tosylsulfonat steht,
mit der Verbindung der Formel III,

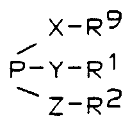

III

in der $R^1$, $R^2$, X, Y und Z die obengenannte Bedeutung haben und
$R^9$ für eine geradkettige oder verzweigte Alkylgruppe mit 1 bis 4 Kohlenstoffatomen steht zur Reaktion gebracht werden.

**2.** Verfahren nach Anspruch 1, dadurch gekennzeichnet, daß man Verbindungen der Formel I worin
$R^1$ und $R^2$ für eine Alkylgruppe mit 1 bis 10 Kohlenstoffatomen, eine Alkenyl- oder Alkinylgruppe mit 2 bis 10 Kohlenstoffatomen, Wasserstoff oder eine Aralkylgruppe mit 7 bis 16 Kohlenstoffatomen stehen, und
$R^3$ und $R^4$ für eine Alkylgruppe mit 1 bis 4 Kohlenstoffatomen, eine Alkenyl- oder Alkinylgruppe mit 2 bis 4 Kohlenstoffatomen oder Wasserstoff stehen,
$R^5$, $R^6$, $R^7$ und $R^8$ für Chlor, Brom, Methoxy oder Wasserstoff stehen und
X, Y und Z für Sauerstoff stehen, herstellt.

**3.** Verfahren zur Herstellung der Verbindung der Formel I nach Anspruch 1, in der R für eine in einen Aldehyd überführbare Gruppe steht, dadurch gekennzeichnet, daß die Verbindung der Formel I, in der R für Aldehyd steht, so umgesetzt wird, daß eine in einen Aldehyd überführbare Gruppe entsteht.

**4.** Verfahren zur Herstellung der Verbindung der Formel I nach Anspruch 1, in der $R^1$ für eine in einen Aldehyd überführbare Gruppe der Formel Ib, Ic oder Id steht,

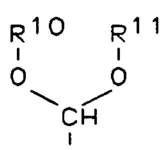

Ib

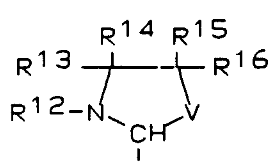

Ic

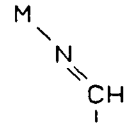

Id

in der $R^{10}$ und $R^{11}$, die gleich oder verschieden sein können, für eine geradkettige oder verzweigte Alkylgruppe mit 1 bis 10 Kohlenstoffatomen stehen oder $R^{10}$ und $R^{11}$ zusammen ein cyclisches Acetal

mit 2 oder 3 Kohlenstoffatomen im Ring bilden,
$R^{12}$ bis $R^{16}$, die gleich oder verschieden sein können, für eine geradkettige oder verzweigte Alkylgruppe mit 1 bis 10 Kohlenstoffatomen oder eine Arylgruppe mit 6, 10 oder 14 Kohlenstoffatomen stehen,
V für Sauerstoff oder Schwefel steht,
M für eine Hydroxygruppe, eine geradkettige oder verzweigte Alkylgruppe mit 1 bis 10 Kohlenstoffatomen, eine Aralkylgruppe mit 7 bis 20 Kohlenstoffatomen, eine Arylgruppe mit 6, 10 oder 14 Kohlenstoffatomen steht oder für einen Rest der Formel Ie oder If,

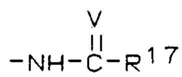

Ie

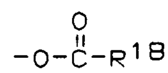

If

in der $R^{17}$ für eine geradkettige oder verzweigte Alkylgruppe mit 1 bis 10 Kohlenstoffatomen, eine Amino-, Pyridin- oder eine Arylgruppe mit 6, 10 oder 14 Kohlenstoffatomen steht und
$R^{18}$ für eine Aminogruppe, eine Pyridingruppe, eine geradkettige oder verzweigte Alkylgruppe mit 1 bis 10 Kohlenstoffatomen, eine Arylgruppe mit 6, 10 oder 14 Kohlenstoffatomen oder eine Aralkylgruppe mit 7 bis 20 Kohlenstoffatomen steht, dadurch gekennzeichnet, daß die Verbindung der Formel I, in der R für eine Aldehydgruppe steht, mit der Verbindung der Formel IVa IVb und/oder IVc, IVd oder IVe,

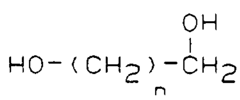

IVa

$R^{10}-OH$

IVb

$R^{11}-OH$

IVc

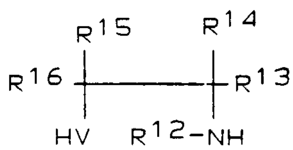

IVd

$M-NH_2$

IVe

in der $R^{10}$ und $R^{11}$ für eine geradkettige oder verzweigte Alkylgruppe mit 1 bis 10 Kohlenstoffatomen stehen,
$R^{12}$ bis $R^{16}$, die gleich oder verschieden sein können, für eine geradkettige oder verzweigte Alkylgruppe mit 1 bis 10 Kohlenstoffatomen oder eine Arylgruppe mit 6, 10 oder 14 Kohlenstoffatomen stehen.
V für Sauerstoff oder Schwefel steht,
M für eine Hydroxygruppe, eine geradkettige oder verzweigte Alkylgruppe mit 1 bis 10 Kohlenstoffatomen, eine Aralkylgruppe mit 7 bis 20 Kohlenstoffatomen, eine Arylgruppe mit 6, 10 oder 14 Kohlenstoffatomen steht oder für einen Rest der Formel Ie oder If,

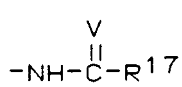

Ie

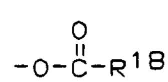

If

in der $R^{17}$ für eine geradkettige oder verzweigte Alkylgruppe mit 1 bis 10 Kohlenstoffatomen, eine

Amino-, Pyridin- oder eine Arylgruppe mit 6, 10 oder 14 Kohlenstoffatomen steht und $R^{18}$ für eine Aminogruppe, eine Pyridingruppe, eine geradkettige oder verzweigte Alkylgruppe mit 1 bis 10 Kohlenstoffatomen, eine Arylgruppe mit 6, 10 oder 14 Kohlenstoffatomen oder eine Aralkylgruppe mit 7 bis 20 Kohlenstoffatomen steht, zur Reaktion gebracht wird.

**5.** Verwendung der Verbindung der Formel I gemäß der Definition in einem oder mehreren der Ansprüche 1 bis 4 zur Herstellung eines Arzneimittels zur Behandlung von Erkrankungen, die durch RNA- oder DNA-Viren hervorgerufen werden.

**6.** Verfahren zur Herstellung einer pharmazeutischen Zubereitung, dadurch gekennzeichnet, daß man mindestens eine Verbindung der Formel I gemäß der Definition in einem oder mehreren der Ansprüche 1 bis 4, gegebenenfalls mit anderen Derivaten der Formel I, mit einem physiologisch annehmbaren Träger sowie gegebenenfalls geeigneten Zusatz- und/oder Hilfsstoffen in eine geeignete Darreichungsform bringt.

**Claims**

**Claims for the following Contracting States : AT, BE, CH, DE, DK, FR, GB, IT, LI, LU, NL, SE**

**1.** A compound of the formula (I)

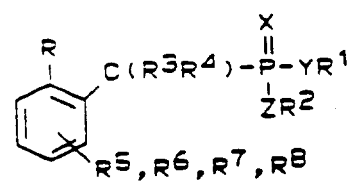

I

in which
R is an aldehyde group or a group which can be converted into an aldehyde,
$R^1$ and $R^2$, which may be the same or different, are a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, hydrogen, sodium, potassium, calcium, magnesium, aluminum, lithium, ammonium or triethylammonium or $R^1$ and $R^2$ together form a cyclic diester having 2 to 6 carbon atoms in the ring,
$R^3$ and $R^4$, which may be the same or different, are a straight-chain or branched alkyl group having 1 to 20 carbon atoms, a straight-chain or branched alkynyl or alkenyl group having 2 to 20 carbon atoms, a cycloalkyl group having 3 to 8 carbon atoms, an alkoxy group having 1 to 4 carbon atoms, hydrogen, fluorine, chlorine, bromine or iodine,
$R^5$, $R^6$, $R^7$ and $R^8$, which may be identical or different, are a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, an alkoxy group having 1 to 4 carbon atoms, hydrogen, fluorine, chlorine, bromine, iodine, a cyanide, hydroxyl or phenyl group or the radical of the formula Ia

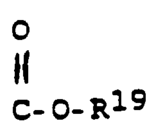

**Ia**

$R^{19}$ is a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, hydrogen, sodium, potassium, calcium, magnesium, aluminum, lithium, ammonium or triethylammonium and
X, Y and Z, which may be identical or different, are oxygen or sulfur
or a prodrug form of the compound of the formula I.

**2.** A compound of the formula I as claimed in claim 1, in which
$R^1$ and $R^2$ are an alkyl group having 1 to 10 carbon atoms, an alkenyl or alkynyl group having 2 to 10 carbon atoms, hydrogen or an aralkyl group having 7 to 16 carbon atoms,
$R^3$ and $R^4$ are an alkyl group having 1 to 4 carbon atoms, an alkenyl or alkynyl group having 2 to 4 carbon atoms or hydrogen,
$R^5$, $R^6$, $R^7$ and $R^8$ are chlorine, bromine, methoxy or hydrogen and
X, Y and Z are oxygen.

**3.** A compound as claimed in claim 1, in which R is a group, which can be converted into an aldehyde, of the formula Ib, Ic or Id

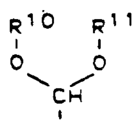
Ib

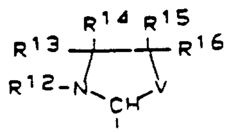
Ic

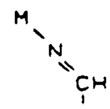
Id

in which
$R^{10}$ and $R^{11}$, which may be identical or different, are a straight-chain or branched alkyl group having 1 to 10 carbon atoms or $R^{10}$ and $R^{11}$ together form a cyclic acetal having 2 or 3 carbon atoms in the ring,
$R^{12}$ to $R^{16}$, which may be identical or different, are a straight-chain or branched alkyl group having 1 to 10 carbon atoms or an aryl group having 6, 10 or 14 carbon atoms,
V is oxygen or sulfur,
M is a hydroxyl group, a straight-chain or branched alkyl group having 1 to 10 carbon atoms, an aralkyl group having 7 to 20 carbon atoms, an aryl group having 6, 10 or 14 carbon atoms or a radical of the formula Ie or If

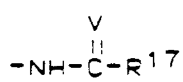
Ie

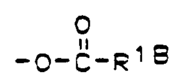
If

in which $R^{17}$ is a straight-chain or branched alkyl group having 1 to 10 carbon atoms, an amino, pyridine or aryl group having 6, 10 or 14 carbon atoms and
$R^{18}$ is an amino group, a pyridine group, a straight-chain or branched alkyl group having 1 to 10 carbon atoms, an aryl group having 6, 10 or 14 carbon atoms or an aralkyl group having 7 to 20 carbon atoms.

**4.** Diethyl 2-formylbenzylphosphonate.

**5.** 2-Formylbenzylphosphonic acid di(triethylammonium) salt.

**6.** Monoethyl 2-formylbenzylphosphonatetriethylammonium salt.

**7.** Diethyl 2-formylbenzylphosphonatethiosemicarbazone.

**8.** A process for the preparation of a compound of the formula I as claimed in claim 1, in which R is an aldehyde group, which comprises reacting a compound of the formula IX

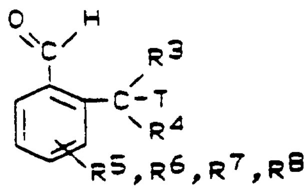

II

in which
$R^3$ and $R^4$, which may be the same or different, are a straight-chain or branched alkyl group having 1 to 20 carbon atoms, a straight-chain or branched alkynyl or alkenyl group having 2 to 20 carbon atoms, a cycloalkyl group having 3 to 8 carbon atoms, an alkoxy group having 1 to 4 carbon atoms, hydrogen, fluorine, chlorine, bromine or iodine,
$R^5$, $R^6$, $R^7$ and $R^8$, which may be identical or different, are a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, an alkoxy group having 1 to 4 carbon atoms, hydrogen, fluorine, chlorine, bromine, iodine, a cyanide, hydroxyl or phenyl group or the radical of the formula Ia

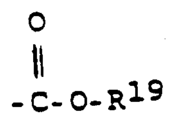

Ia

$R^{19}$ is a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, hydrogen, sodium, potassium, calcium, magnesium, aluminum, lithium, ammonium or triethylammonium and
T is chlorine, bromine, iodine, methylsulfonate, phenylsulfonate or tosylsulfonate, with a compound of the formula III

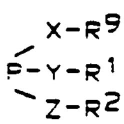

III

in which,
$R^1$ and $R^2$, which may be the same or different, are a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, hydrogen, sodium, potassium, calcium, magnesium, aluminum, lithium, ammonium or triethylammonium or $R^1$ and $R^2$ together form a cyclic diester having 2 to 6 carbon atoms in the ring,
$R^9$ is a straight-chain or branched alkyl group having 1 to 4 carbon atoms and
X, Y and Z, which may be identical or different, are oxygen or sulfur.

**9.** A process for the preparation of a compound of the formula I as claimed in claim 1, in which R is a group which can be converted into an aldehyde, which comprises reacting a compound of the formula I in which R is aldehyde in such a way that a group which can be converted into an aldehyde is formed.

**10.** A process for the preparation of a compound of the formula I as claimed in claim 3, which comprises reacting a compound of the formula I in which R is an aldehyde group with a compound of the formula IVa, IVb and/or IVc, IVd or IVe

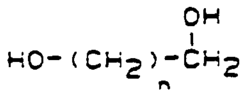

IVa

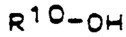

IVb

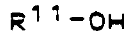

IVc

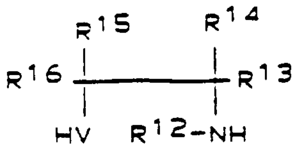

IVd

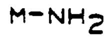

IVe

in which $R^{10}$ and $R^{11}$ are a straight-chain or branched alkyl group having 1 to 10 carbon atoms,
$R^{12}$ to $R^{16}$, which may be identical or different, are a straight-chain or branched alkyl group having 1 to 10 carbon atoms or an aryl group having 6, 10 or 14 carbon atoms,
V is oxygen or sulfur,
M is a hydroxyl group, a straight-chain or branched alkyl group having 1 to 10 carbon atoms, an aralkyl group having 7 to 20 carbon atoms, an aryl group having 6, 10 or 14 carbon atoms or a radical of the formula Ie or If

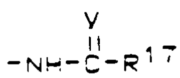

Ie

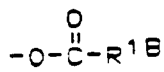

If

in which $R^{17}$ is a straight-chain or branched alkyl group having 1 to 10 carbon atoms, or an amino, pyridine, or an aryl group having 6, 10 or 14 carbon atoms and
$R^{18}$ is an amino group, a pyridine group, a straight-chain or branched alkyl group having 1 to 10 carbon atoms, an aryl group having 6, 10 or 14 carbon atoms or an aralkyl group having 7 to 20 carbon atoms.

**11.** The use of a compound of the formula I as claimed in claim 1 for the production of a pharmaceutical for the treatment of diseases which are caused by RNA or DNA viruses.

**12.** The use of a compound of the formula I as claimed in claim 2 for the production of a pharmaceutical for the treatment of diseases which are caused by RNA or DNA viruses.

**13.** The use of a compound of the formula I as claimed in claim 3 for the production of a pharmaceutical for the treatment of diseases which are caused by RNA or DNA viruses.

**14.** A pharmaceutical preparation containing an effective amount of a compound as claimed in claim 1.

**15.** A method for the production of a pharmaceutical, wherein an effective amount of a compound as claimed in claim 1 is converted into a suitable form for administration.

**Claims for the following Contracting States : ES, GR**

**1.** A process for the preparation of a compound of the formula (I)

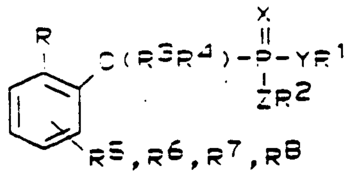

I

in which
R is an aldehyde group,
$R^1$ and $R^2$, which may be the same or different, are a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, hydrogen, sodium, potassium, calcium, magnesium, aluminum, lithium, ammonium or triethylammonium or $R^1$ and $R^2$ together form a cyclic diester having 2 to 6 carbon atoms in the ring,
$R^3$ and $R^4$, which may be the same or different, are a straight-chain or branched alkyl group having 1 to 20 carbon atoms, a straight-chain or branched alkynyl or alkenyl group having 2 to 20 carbon atoms, a cycloalkyl group having 3 to 8 carbon atoms, an alkoxy group having 1 to 4 carbon atoms, hydrogen, fluorine, chlorine, bromine or iodine,
$R^5$, $R^6$, $R^7$ and $R^8$, which may be identical or different, are a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, an alkoxy group having 1 to 4 carbon atoms, hydrogen, fluorine, chlorine, bromine, iodine, a cyanide, hydroxyl or phenyl group or the radical of the formula Ia

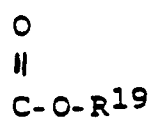

Ia

$R^{19}$ is a straight-chain or branched alkyl group having 1 to 20 carbon atoms, a straight-chain or branched alkenyl or alkynyl group having 2 to 20 carbon atoms, an aralkyl group having 7 to 20 carbon atoms, a cycloalkyl group having 3 to 8 carbon atoms, hydrogen, sodium, potassium, calcium, magnesium, aluminum, lithium, ammonium or triethylammonium and
X, Y and Z, which may be identical or different, are oxygen or sulfur which comprises reacting a compound of the formula II

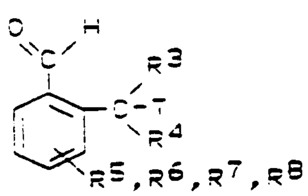

II

in which
$R^3$, $R^4$, $R^5$, $R^6$, $R^7$ and $R^8$ are as defined above and T is chlorine, bromine, iodine, methylsulfonate, phenylsulfonate or tosylsulfonate,
with a compound of the formula III

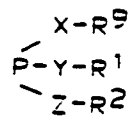

III

in which,

$R^1$, $R^2$, X, Y and Z are as defined above and $R^9$ is a straight-chain or branched alkyl group having 1 to 4 carbon atoms.

**2.** The process as claimed in claim 1, which comprises preparing compounds of the formula I in which
$R^1$ and $R^2$ are an alkyl group having 1 to 10 carbon atoms, an alkenyl or alkynyl group having 2 to 10 carbon atoms, hydrogen or an aralkyl group having 7 to 16 carbon atoms,
$R^3$ and $R^4$ are an alkyl group having 1 to 4 carbon atoms, an alkenyl or alkynyl group having 2 to 4 carbon atoms or hydrogen,
$R^5$, $R^6$, $R^7$ and $R^8$ are chlorine, bromine, methoxy or hydrogen and
X, Y and Z are oxygen.

**3.** The process for the preparation of the compound of the formula I as claimed in claim 1, in which R is a group which can be converted into an aldehyde, which comprises reacting the compound of the formula I in which R is aldehyde in such a way that a group which can be converted into an aldehyde is formed.

**4.** A process for the preparation of a compound of the formula I as claimed in claim 1, in which $R^1$ is a group, which can be converted into an aldehyde, of the formula Ib, Ic or Id

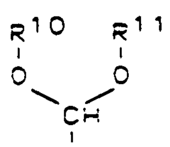

Ib

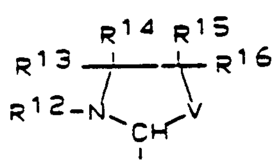

Ic

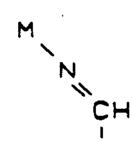

Id

in which
$R^{10}$ and $R^{11}$, which may be identical or different, are a straight-chain or branched alkyl group having 1 to 10 carbon atoms or $R^{10}$ and $R^{11}$ together form a cyclic acetal having 2 or 3 carbon atoms in the ring,
$R^{12}$ to $R^{16}$, which may be identical or different, are a straight-chain or branched alkyl group having 1 to 10 carbon atoms or an aryl group having 6, 10 or 14 carbon atoms,
V is oxygen or sulfur,
M is a hydroxyl group, a straight-chain or branched alkyl group having 1 to 10 carbon atoms, an aralkyl group having 7 to 20 carbon atoms, an aryl group having 6, 10 or 14 carbon atoms or a radical of the formula Ie or If

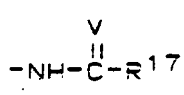

Ie

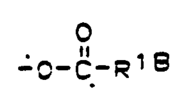

If

in which $R^{17}$ is a straight-chain or branched alkyl group having 1 to 10 carbon atoms, an amino, pyridine, or aryl group having 6, 10 or 14 carbon atoms and
$R^{18}$ is an amino group, a pyridine group, a straight-chain or branched alkyl group having 1 to 10 carbon atoms, an aryl group having 6, 10 or 14 carbon atoms or an aralkyl group having 7 to 20 carbon atoms
which comprises reacting a compound of the formula I in which R is an aldehyde group with a

compound of the formula IVa, IVb and/or IVc, IVd or IVe

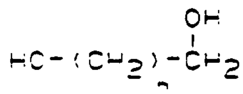 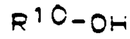 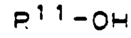

IVa IVb IVc

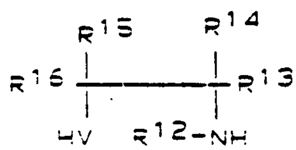 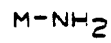

IVd IVe

in which $R^{10}$ and $R^{11}$ are a straight-chain or branched alkyl group having 1 to 10 carbon atoms,
$R^{12}$ to $R^{16}$, which may be identical or different, are a straight-chain or branched alkyl group having 1 to 10 carbon atoms or an aryl group having 6, 10 or 14 carbon atoms,
V is oxygen or sulfur,
M is a hydroxyl group, a straight-chain or branched alkyl group having 1 to 10 carbon atoms, an aralkyl group having 7 to 20 carbon atoms, an aryl group having 6, 10 or 14 carbon atoms or a radical of the formula Ie or If

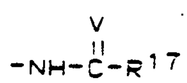 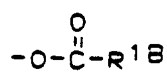

Ie If

in which $R^{17}$ is a straight-chain or branched alkyl group having 1 to 10 carbon atoms, or an amino, pyridine, or an aryl group having 6, 10 or 14 carbon atoms and
$R^{18}$ is an amino group, a pyridine group, a straight-chain or branched alkyl group having 1 to 10 carbon atoms, an aryl group having 6, 10 or 14 carbon atoms or an aralkyl group having 7 to 20 carbon atoms.

**5.** The use of a compound of the formula I as defined in one or more of claims 1 to 4 for the production of a pharmaceutical for the treatment of diseases which are caused by RNA or DNA viruses.

**6.** A method for the production of a pharmaceutical preparation, which comprises bringing at least one compound of the formula I as defined in one or more claims 1 to 4 into a suitable form for administration, if desired with other derivatives of the formula I, with a physiologically acceptable excipient and, if desired, with appropriate additives and/or auxiliaries.

**Revendications**

**Revendications pour les Etats contractants suivants : AT, BE, CH, DE, DK, FR, GB, IT, LI, LU, NL, SE**

**1.** Composé de formule I

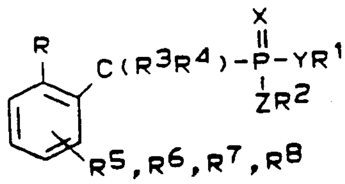

I

dans laquelle

| | |
|---|---|
| R | représente un groupe aldéhyde ou un groupe pouvant être converti en un aldéhyde, |
| $R^1$ et $R^2$, | qui peuvent être identiques ou différents, représentent un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un atome d'hydrogène, de sodium, de potassium, de calcium, de magnésium, d'aluminium, de lithium, l'ion ammonium ou triéthylammonium, ou $R^1$ et $R^2$ forment ensemble un diester cyclique ayant de 2 à 6 atomes de carbone dans le cycle, |
| $R^3$ et $R^4$, | qui peuvent être identiques ou différents, représentent un groupe alkyle à chaîne droite ou ramifiée ayant de 1 à 20 atomes de carbone, un groupe alcynyle ou alcényle à chaîne droite ou ramifiée ayant de 2 à 20 atomes de carbone, un groupe cycloalkyle ayant de 3 à 8 atomes de carbone, un groupe alcoxy ayant de 1 à 4 atomes de carbone, ou un atome d'hydrogène, de fluor, de chlore, de brome ou d'iode, |
| $R^5$, $R^6$, $R^7$ et $R^8$, | qui peuvent être identiques ou différents, représentent un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un groupe alcoxy ayant de 1 à 4 atomes de carbone, ou un atome d'hydrogène, de fluor, de chlore, de brome ou d'iode, ou le groupe cyano, hydroxy ou phényle, ou un radical de formule Ia |

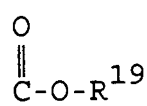

Ia

| | |
|---|---|
| $R^{19}$ | représentant un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone ou un atome d'hydrogène, de sodium, de potassium, de calcium, de magnésium, d'aluminium ou de lithium, ou l'ion ammonium ou triéthylammonium, et |
| X, Y et Z, | qui peuvent être identiques ou différents, représentent un atome d'oxygène ou de soufre, |

ou forme de précurseur de médicament du composé de formule I.

**2.** Composé de formule I selon la revendication 1, dans lequel

| | |
|---|---|
| $R^1$ et $R^2$ | représentent un groupe alkyle ayant de 1 à 10 atomes de carbone, un groupe alcényle ou acynyle ayant de 2 à 10 atomes de carbone, un atome d'hydrogène ou un groupe aralkyle ayant de 7 à 16 atomes de carbone, |
| $R^3$ et $R^4$ | représentent un groupe alkyle ayant de 1 à 4 atomes de carbone, un groupe |

| | |
|---|---|
| | alcényle ou alcynyle ayant de 2 à 4 atomes de carbone, ou un atome d'hydrogène, |
| $R^5$, $R^6$, $R^7$ et $R^8$ | représentent un atome d'hydrogène, de chlore ou de brome ou le groupe méthoxy, et |
| X, Y et Z | représentent un atome d'oxygène. |

**3.** Composé selon la revendication 1, dans lequel R représente un groupe de formule Ib, Ic ou Id, pouvant être converti en aldéhyde,

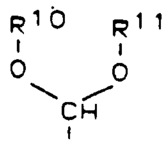

Ib

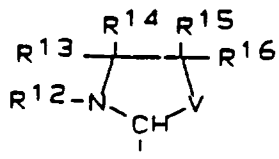

Ic

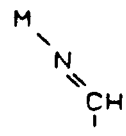

Id

formules dans lesquelles

| | |
|---|---|
| $R^{10}$ et $R^{11}$, | qui peuvent être identiques ou différents, représentent un radical alkyle à chaîne droite ou ramifiée ayant de 1 à 10 atomes de carbone, ou $R^{10}$ et $R^{11}$ forment ensemble un acétal cyclique ayant 2 ou 3 atomes de carbone dans le cycle, |
| $R^{12}$ à $R^{16}$, | qui peuvent être identiques ou différents, représentent un groupe alkyle à chaîne droite ou ramifiée ayant de 1 à 10 atomes de carbone ou un groupe aryle ayant 6, 10 ou 14 atomes de carbone, |
| V | représente un atome d'oxygène ou de soufre, |
| M | représente le groupe hydroxy, un groupe alkyle à chaîne droite ou ramifiée ayant de 1 à 10 atomes de carbone, un groupe aralkyle ayant de 7 à 20 atomes de carbone, un groupe aryle ayant 6, 10 ou 14 atomes de carbone, ou un radical de formule Ie ou If |

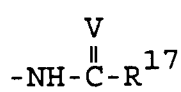

Ie

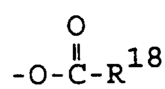

If

formules dans lesquelles

| | |
|---|---|
| $R^{17}$ | représente un groupe alkyle à chaîne droite ou ramifiée ayant de 1 à 10 atomes de carbone, un groupe amino, pyridine ou un groupe aryle ayant 6, 10 ou 14 atomes de carbone, et |
| $R^{18}$ | représente un groupe amino, un groupe pyridine, un groupe alkyle à chaîne droite ou ramifiée ayant de 1 à 10 atomes de carbone, un groupe aryle ayant 6, 10 ou 14 atomes de carbone ou un groupe aralkyle ayant de 7 à 20 atomes de carbone. |

**4.** 2-formylbenzylphosphonate de diéthyle.

**5.** 2-formylbenzylphosphonate de ditriéthylammonium.

**6.** 2-formylbenzylphosphonate de monoéthyle et de triéthylammonium .

**7.** 2-formylbenzylphosphonate de diéthyl-thiosemicarbazone.

**8.** Procédé pour la préparation d'un composé de formule I selon la revendication 1, dans lequel R représente un groupe aldéhyde, caractérisé en ce que l'on met en réaction un composé de formule II

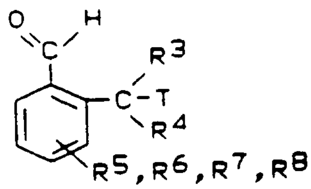

II

dans laquelle

R³ et R⁴, qui peuvent être identiques ou différents, représentent un groupe alkyle à chaîne droite ou ramifiée ayant de 1 à 20 atomes de carbone, un groupe alcynyle ou alcényle à chaîne droite ou ramifiée ayant de 2 à 20 atomes de carbone, un groupe cycloalkyle ayant de 3 à 8 atomes de carbone, un groupe alcoxy ayant de 1 à 4 atomes de carbone, un atome d'hydrogène ou de fluor, de chlore, de brome ou d'iode,

$R^5$, $R^6$, $R^7$ et $R^8$, qui peuvent être identiques ou différents, représentent un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un groupe alcoxy ayant de 1 à 4 atomes de carbone, un atome d'hydrogène, de fluor, de chlore, de brome, d'iode, le groupe cyano, hydroxy ou phényle, ou un radical de formule Ia,

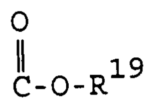

Ia

$R^{19}$ représentant un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un atome d'hydrogène, de sodium, de potassium, de calcium, de magnésium, d'aluminium, de lithium, l'ion ammonium ou triéthylammonium, et

T représente un atome de chlore, de brome ou d'iode, ou le groupe méthylsulfonate, phénylsulfonate ou tosylsulfonate,

avec un composé de formule III

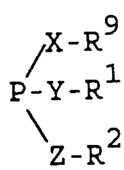

III

dans laquelle

$R^1$ et $R^2$, qui peuvent être identiques ou différents, représentent un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un atome d'hydrogène, de sodium, de potassium, de calcium, de magnésium, d'aluminium, de lithium, l'ion ammonium ou triéthylammonium, ou $R^1$ et $R^2$ forment ensemble un diester cyclique ayant de 2 à 6 atomes de carbone dans le cycle,

$R^9$ représente un groupe alkyle à chaîne droite ou ramifiée ayant de 1 à 4 atomes de carbone, et

X, Y et Z, qui peuvent être identiques ou différents, représentent un atome d'oxygène ou de soufre.

**9.** Procédé pouur la préparation d'un composé de formule I selon la revendication 1, dans lequel R représente un groupe pouvant être converti en un aldéhyde, caractérisé en ce que l'on fait réagir un composé de formule I dans lequel R représente un aldéhyde, de manière à obtenir un groupe pouvant être converti en un aldéhyde.

**10.** Procédé pour la préparation d'un composé de formule I selon la revendication 3, caractérisé en ce que l'on met en réaction un composé de formule I, dans lequel R représente un groupe aldéhyde, avec un composé de formule IVa, IVb et/ou IVc, IVd ou IVe

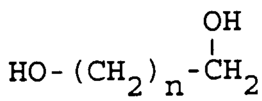

IVa

$R^{10}$-OH

IVb

$R^{11}$-OH

IVc

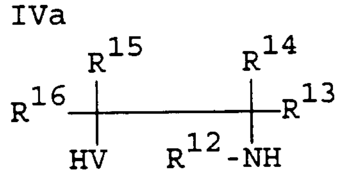

IVd

M-$NH_2$

IVe

formules dans lesquelles $R^{10}$ et $R^{11}$ représentent un groupe alkyle à chaîne droite ou ramifiée ayant de 1 à 10 atomes de carbone, $R^{12}$ à $R^{16}$, qui peuvent être identiques ou différents, représentent un groupe alkyle à chaîne droite ou ramifiée ayant de 1 à 10 atomes de carbone ou un groupe aryle ayant 6, 10 ou 14 atomes de carbone,

V représente un atome d'oxygène ou de soufre,

M représente le groupe hydroxy ou un groupe alkyle à chaîne droite ou ramifiée ayant de 1 à 10 atomes de carbone, un groupe aralkyle ayant de 7 à 20 atomes de carbone, un groupe aryle ayant 6, 10 ou 14 atomes de carbone, ou un radical de formule Ie ou If

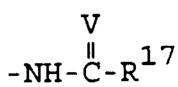

Ie

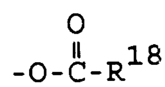

If

formules dans lesquelles

$R^{17}$ représente un groupe alkyle à chaîne droite ou ramifiée ayant de 1 à 10 atomes de carbone, un groupe amino, pyridine ou un groupe aryle ayant 6, 10 ou 14 atomes de carbone, et

$R^{18}$ représente un groupe amino, un groupe pyridine, un groupe alkyle à chaîne droite ou ramifiée ayant de 1 à 10 atomes de carbone, un groupe aryle ayant 6, 10 ou 14 atomes de carbone ou un groupe aralkyle ayant de 7 à 20 atomes de carbone.

**11.** Utilisation d'un composé de formule I selon la revendication 1, pour la fabrication d'un médicament destiné au traitement de maladies qui sont provoquées par des virus à ARN ou à ADN.

**12.** Utilisation d'un composé de formule I selon la revendication 2, pour la fabrication d'un médicament destiné au traitement de maladies qui sont provoquées par des virus à ARN ou à ADN.

**13.** Utilisation d'un composé de formule I selon la revendication 3, pour la fabrication d'un médicament destiné au traitement de maladies qui sont provoquées par des virus à ARN ou à ADN.

**14.** Compositions pharmaceutiques contenant une quantité efficace d'un composé selon la revendication 1.

**15.** Procédé pour la fabrication d'un médicament, caractérisé en ce que l'on met sous une forme d'administration appropriée une quantité efficace d'un composé selon la revendication 1.

**Revendications pour les Etats contractants suivants : ES, GR**

**1.** Procédé pour la préparation d'un composé de formule I

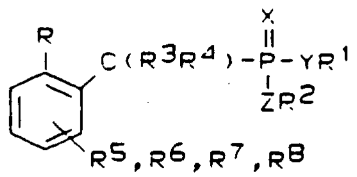

I

dans laquelle

R représente un groupe aldéhyde ou un groupe pouvant être converti en un aldéhyde,

$R^1$ et $R^2$, qui peuvent être identiques ou différents, représentent un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un atome d'hydrogène, de sodium, de potassium, de calcium, de magnésium, d'aluminium, de lithium, l'ion ammonium ou triéthylammonium, ou $R^1$ et $R^2$ forment ensemble un diester cyclique ayant de 2 à 6 atomes de carbone dans le cycle,

$R^3$ et $R^4$, qui peuvent être identiques ou différents, représentent un groupe alkyle à chaîne droite ou ramifiée ayant de 1 à 20 atomes de carbone, un groupe alcynyle ou alcényle à chaîne droite ou ramifiée ayant de 2 à 20 atomes de carbone, un groupe cycloalkyle ayant de 3 à 8 atomes de carbone, un groupe alcoxy ayant de 1 à 4 atomes de carbone, ou un atome d'hydrogène, de fluor, de chlore, de brome ou d'iode,

$R^5$, $R^6$, $R^7$ et $R^8$, qui peuvent être identiques ou différents, représentent un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone, un groupe alcoxy ayant de 1 à 4 atomes de carbone, ou un atome d'hydrogène, de fluor, de chlore, de brome ou d'iode, ou le groupe cyano, hydroxy ou phényle, ou un radical de formule Ia

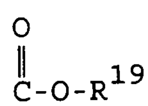

Ia

$R^{19}$ représentant un groupe alkyle à chaîne droite ou ramifiée ayant de 1 à 20 atomes de carbone, un groupe alcényle ou alcynyle à chaîne droite ou ramifiée ayant de 2 à 20 atomes de carbone, un groupe aralkyle ayant de 7 à 20 atomes de carbone, un groupe cycloalkyle ayant de 3 à 8 atomes de carbone ou un atome d'hydrogène, de sodium, de potassium, de calcium, de magnésium, d'aluminium ou de lithium, ou l'ion ammonium ou triéthylammonium, et

X, Y et Z, qui peuvent être identiques ou différents, représentent un atome d'oxygène ou de soufre,

caractérisé en ce que l'on met en réaction un composé de formule II

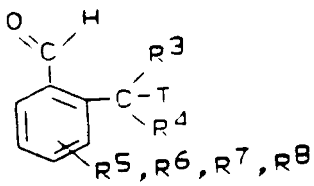

II

dans laquelle

| | |
|---|---|
| $R^3$, $R^4$, $R^5$, $R^6$, $R^7$ et $R^8$ | ont les significations données ci-dessus, et |
| T | représente un atome de chlore, de brome ou d'iode ou le groupe méthylsulfonate, phénylsulfonate ou tosylsulfonate, |

avec un composé de formule III

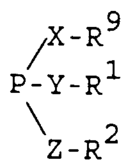

III

dans laquelle

| | |
|---|---|
| $R^1$, $R^2$, X, Y et Z | ont les significations données ci-dessus, et |
| $R^9$ | représente un groupe alkyle à chaîne droite ou ramifiée ayant de 1 à 4 atomes de carbone. |

**2.** Procédé selon la revendication 1, caractérisé en ce que l'on prépare des composés de formule I dans lesquels

| | |
|---|---|
| $R^1$ et $R^2$ | représentent un groupe alkyle ayant de 1 à 10 atomes de carbone, un groupe alcényle ou acynyle ayant de 2 à 10 atomes de carbone, un atome d'hydrogène ou un groupe aralkyle ayant de 7 à 16 atomes de carbone, |
| $R^3$ et $R^4$ | représentent un groupe alkyle ayant de 1 à 4 atomes de carbone, un groupe alcényle ou alcynyle ayant de 2 à 4 atomes de carbone, ou un atome d'hydrogène, |
| $R^5$, $R^6$, $R^7$ et $R^8$ | représentent un atome d'hydrogène, de chlore ou de brome ou le groupe méthoxy, et |
| X, Y et Z | représentent un atome d'oxygène. |

**3.** Procédé pour la préparation d'un composé de formule I selon la revendication 1, dans lequel R représente un groupe pouvant être converti en un aldéhyde, caractérisé en ce que l'on fait réagir un composé de formule I, dans lequel R représente un aldéhyde, de manière à obtenir un groupe pouvant être converti en un aldéhyde.

**4.** Procédé pour la préparation d'un composé de formule I selon la revendication 1, dans lequel R représente un groupe de formule Ib, Ic ou Id, pouvant être converti en aldéhyde,

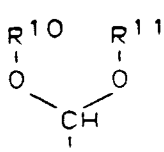

Ib

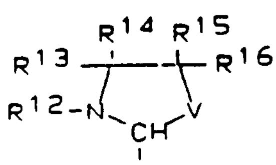

Ic

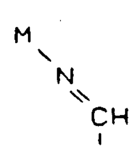

Id

formules dans lesquelles

- $R^{10}$ et $R^{11}$, qui peuvent être identiques ou différents, représentent un radical alkyle à chaîne droite ou ramifiée ayant de 1 à 10 atomes de carbone, ou $R^{10}$ et $R^{11}$ forment ensemble un acétal cyclique ayant 2 ou 3 atomes de carbone dans le cycle,
- $R^{12}$ à $R^{16}$, qui peuvent être identiques ou différents, représentent un groupe alkyle à chaîne droite ou ramifiée ayant de 1 à 10 atomes de carbone ou un groupe aryle ayant 6, 10 ou 14 atomes de carbone,
- V représente un atome d'oxygène ou de soufre,
- M représente le groupe hydroxy, un groupe alkyle à chaîne droite ou ramifiée ayant de 1 à 10 atomes de carbone, un groupe aralkyle ayant de 7 à 20 atomes de carbone, un groupe aryle ayant 6, 10 ou 14 atomes de carbone, ou un radical de formule Ie ou If

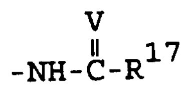
Ie

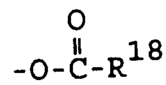
If

formules dans lesquelles

- $R^{17}$ représente un groupe alkyle à chaîne droite ou ramifiée ayant de 1 à 10 atomes de carbone, un groupe amino, pyridine ou un groupe aryle ayant 6, 10 ou 14 atomes de carbone, et
- $R^{18}$ représente un groupe amino, un groupe pyridine, un groupe alkyle à chaîne droite ou ramifiée ayant de 1 à 10 atomes de carbone, un groupe aryle ayant 6, 10 ou 14 atomes de carbone ou un groupe aralkyle ayant de 7 à 20 atomes de carbone,

caractérisé en ce que l'on met en réaction un composé de formule I, dans lequel R représente un groupe aldéhyde, avec un composé de formule IVa, IVb et/ou IVc, IVd ou IVe

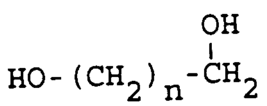
IVa

$R^{10}$-OH
IVb

$R^{11}$-OH
IVc

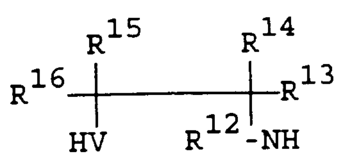
IVd

M-$NH_2$
IVe

formules dans lesquelles $R^{10}$ et $R^{11}$ représentent un groupe alkyle à chaîne droite ou ramifiée ayant de 1 à 10 atomes de carbone, $R^{12}$ à $R^{16}$, qui peuvent être identiques ou différents, représentent un groupe alkyle à chaîne droite ou ramifiée ayant de 1 à 10 atomes de carbone ou un groupe aryle ayant 6, 10 ou 14 atomes de carbone,

- V représente un atome d'oxygène ou de soufre,
- M représente le groupe hydroxy ou un groupe alkyle à chaîne droite ou ramifiée ayant de 1 à 10 atomes de carbone, un groupe aralkyle ayant de 7 à 20 atomes de carbone, un groupe aryle ayant 6, 10 ou 14 atomes de carbone, ou un radical de formule Ie ou If

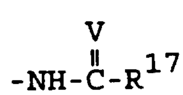
Ie

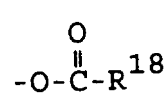
If

formules dans lesquelles

$R^{17}$ représente un groupe alkyle à chaîne droite ou ramifiée ayant de 1 à 10 atomes de carbone, un groupe amino, pyridine ou un groupe aryle ayant 6, 10 ou 14 atomes de carbone, et

$R^{18}$ représente un groupe amino, un groupe pyridine, un groupe alkyle à chaîne droite ou ramifiée ayant de 1 à 10 atomes de carbone, un groupe aryle ayant 6, 10 ou 14 atomes de carbone ou un groupe aralkyle ayant de 7 à 20 atomes de carbone.

**5.** Utilisation d'un composé de formule I selon la définition donnée dans une ou plusieurs des revendications 1 à 4, pour la fabrication d'un médicament destiné au traitement de maladies qui sont provoquées par des virus à ARN ou à ADN.

**6.** Procédé pour la préparation d'une composition pharmaceutique, caractérisé en ce que l'on met sous une forme d'administration appropriée au moins un composé de formule I selon la définition donnée dans une ou plusieurs des revendications 1 à 4, éventuellement avec d'autres dérivés de formule I, conjointement avec un véhicule physiologiquement acceptable ainsi qu'éventuellement des additifs et/ou adjuvants appropriés.